(19) Europäisches Patentamt
European Patent Office
Office européen des brevets

(11) Publication number : **0 336 475 B1**

# (12) EUROPEAN PATENT SPECIFICATION

(45) Date of publication of patent specification : **23.12.92 Bulletin 92/52**

(51) Int. Cl.$^5$ : **C07D 487/10,** C08G 73/06, C08G 59/62, // (C07D487/10, 209:00, 209:00)

(21) Application number : **89200725.3**

(22) Date of filing : **21.03.89**

(54) **1,6-diazaspiro (4,4)nonane-2,7-dione derivatives.**

(30) Priority : **23.03.88 US 171998
23.03.88 US 172000
23.03.88 US 172052
23.03.88 US 172054
30.03.88 US 175023
25.04.88 US 185574**

(43) Date of publication of application : **11.10.89 Bulletin 89/41**

(45) Publication of the grant of the patent : **23.12.92 Bulletin 92/52**

(84) Designated Contracting States : **BE CH DE ES FR GB IT LI NL**

(56) References cited :
**EP-A- 0 106 206
US-A- 4 672 102
PATENT ABSTRACTS OF JAPAN vol. 9, no. 188 (C-295)(1911), 3rd August 1985**

(73) Proprietor : **SHELL INTERNATIONALE RESEARCH MAATSCHAPPIJ B.V.
Carel van Bylandtlaan 30
NL-2596 HR Den Haag (NL)**

(72) Inventor : **Wang, Pen Chung
13603 Pinerock Lane
Houston, Texas 77079 (US)**

EP 0 336 475 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to the production of certain novel 1,6-diazaspiro[4,4]nonane-2,7-dione derivatives. More particularly, the invention relates to certain novel 1,6-di(oxyphenyl)-1,6-diazaspiro[4,4]nonane-2,7-dione derivatives.

Reaction products of epichlorohydrin and spirobiindanols are disclosed in U.S. 4,672,102, wherein the resulting polyhydric polyethers are said to have high heat distortion temperatures. The values reported are from about 131°C to about 153°C, depending upon the nature of the substituents present. The corresponding value for the polyhydric polyether of 2,2-di(4-hydroxyphenyl)propane was 88°C. It would be of advantage to provide a class of novel starting materials, being or leading to phenoxy-type resins having comparable or even higher glass transition temperatures.

Accordingly Applicants have prepared novel 1,6-diazaspiro[4,4]nonane-2,7-dione derivatives having the formula $X-\{-B-Y-(P-Y)_p-\}_m-B-X$, in which:
B is a divalent radical of formula (I),

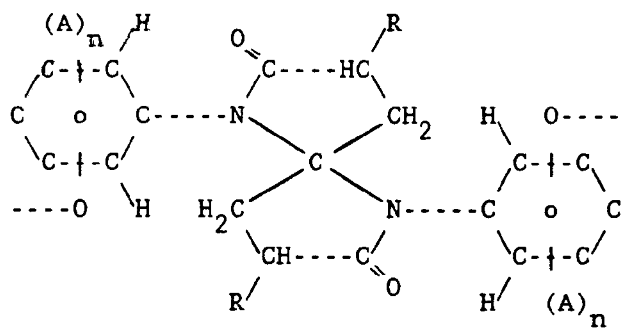

(I)

wherein R independently is hydrogen or a $C_{1-4}$ alkyl, A is a substituent replacing hydrogen selected independently from a $C_{1-4}$ alkyl or halogen atom, and n independently is 0, 1 or 2; $m \geqq 0$; each X independently is hydrogen or a glycidyl group; Y is 2-hydroxy-1,3-propadiyl; each P independently is a divalent radical derived from a dihydric aromatic alcohol; and p is on average of from 0 to 3. A preferably is methyl.

The difunctional, polycyclic hydroxyphenyl substituted 1,6-diazaspiro[4,4]nonane-2,7-diones of the invention in which m is 0, are alternatively termed dihydric spirodilactams and can be prepared by reacting in liquid phase solution an aminophenol of formula (II),

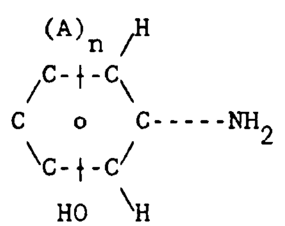

(II)

wherein A and n have the same meaning as defined before, with either (reactant IIa) a 4-oxoheptanedioic acid compound of formula (III),

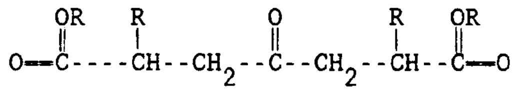

(III)

or (reactant IIb) a 1,6-dioxaspiro[4,4]nonane-2,7-dione of formula (IV),

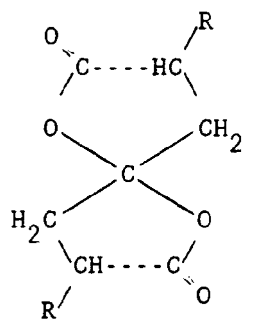

(IV)

wherein R for both reactants has the same meaning as defined before. Illustrative dihydric spirodilactams include 1,6-di(4-hydroxyphenyl)-1,6-diazaspiro[4,4]nonane-2,7-dione, 1,6-di(3-hydroxyphenyl)-1,6-diazaspiro [4.4]nonane-2,7-dione, 1,6-di(4-hydroxy-3-methylphenyl)-1,6-diazaspiro[4.4]nonane-2,7-dione, 1,6-di(3-hydroxyphenyl)-3,8-dimethyl-1,6-diazaspiro[4.4]nonane-2,7-dione, 1,6-di(4-hydroxy-3-methylphenyl)-3,8-dimethyl-1,6-diazaspiro[4.4]nonane-2,7-dione.Of the dihydric spirodilactams of the above formula, 1,6-di(4-hydroxyphenyl)-1,6-diazaspiro[4.4]nonane-2,7-dione is particularly preferred.

The corresponding glycidyloxyphenyl-substituted spirodilactams are produced from the dihydric spirodilactams by conventional technology typically employed for the production of glycidyloxyphenyl-containing epoxy compounds.

Preferably the dihydric or glycidyloxyphenyl substituted spirodilactam is represented by the formula wherein R is hydrogen. Particularly preferred are spirodilactams formula (I) wherein n is 0, spirodilactams wherein n is 0 and R is hydrogen being most preferred. The X-oxyphenyl substituent may be a 3- or a 4-X-oxyphenyl substituent, the latter being most preferred.

The reaction of the 4-oxoheptanedioic acid of formula (III) or its (di)alkylester (i.e., compound IIa), and the aminophenol is referred to as "route A", the reaction of the 1,6-dioxaspiro[4,4]nonane-2,7-dione of formula (IV) (compound IIb) and the aminophenol respectively "route B".

Illustrative aminophenols include 4-aminophenol, 3-aminophenol, 3-amino-6-methylphenol, 4-amino-2-bromophenol, 4-amino-2,6-dibromophenol, 4-amino-2-chlorophenol, 3-amino-5-ethylphenol and 4-amino-2-chloro-6-methylphenol. The otherwise unsubstituted aminophenols, i.e., 4-aminophenol and 3-aminophenol, are the preferred reactants and 4-aminophenol is particularly preferred.

Illustrative 4-oxoheptanedioic acid compounds include 4-oxoheptanedioic acid, dimethyl 4-oxoheptanedioate, diethyl 2,6-dimethyl-4-oxoheptanedioate, dipropyl 2-methyl-4-oxoheptanedioate, 2,6-dimethyl-4-oxoheptanedioic acid and the mono-methyl ester of 4-oxoheptanedioic acid. In general, the preferred 4-oxoheptanedioic acid compounds are those of the above formula wherein each R is hydrogen or methyl and particularly preferred are 4-oxoheptanedioic acid or dimethyl 4-oxoheptanedioate.

Route B uses a 1,6-dioxaspiro[4.4]nonane-2,7-dione of formula (IV). Such "spirodilactones" are produced from analogous unsubstituted or substituted succinic anhydrides. Illustrative spirodilactones useful in the process of the invention are 1,6-dioxaspiro-[4.4]nonane-2,7-dione, 3,8-dimethyl-1,6-dioxaspiro[4.4]nonane-2,7-dione, 3,8-diethyl-1,6-dioxaspiro[4.4]nonane-2,7-dione and 3-methyl-1,6-dioxaspiro[4.4]nonane-2,7-dione. The spirodilactone free from alkyl ring substituents, i.e., 1,6-dioxaspiro[4.4]nonane-2,7-dione, is the more preferred spirodilactone reactant.

In the process of the invention the aminophenol is employed in an amount of from 1 mole to 5 moles per mole of reactant II (i.e., for route A the 4-oxoheptanedioic acid compound, and for route B the spirodilactone) but preferably in an amount of from 1.5 mole to 3 moles per mole of reactant II.

The reaction of reactant II and the aminophenol takes place in liquid phase solution in a liquid reaction diluent. Suitable reaction diluents are liquid under reaction conditions and are polar diluents in which reactant II and aminophenol are soluble, at least at reaction temperature, and which are inert to the reactants and the dihydric spirodilactam product. Suitable diluents include dialkyl ketones such as methyl ethyl ketone, methyl isobutyl ketone and diisopropyl ketone; esters such as butyl acetate and methyl 2-ethylhexanoate; ethers including acyclic ethers such as diethylene glycol dimethyl ether and triethylene glycol diethyl ether as well as cyclic ethers such as tetrahydrofuran and dioxane; N,N-dialkylamides such as N,N-dimethylacetamide, N,N-dimethylformamide and N,N-diethylacetamide and sulfur-containing diluents such as dimethyl sulfoxide and sulfolane. Of these diluent types, the N,N-dialkylamides are a preferred class, particularly N,N-dimethylacetamide.

Reactant II and the aminophenol are contacted under reaction conditions in solution in the reaction diluent by conventional methods such as shaking, stirring or refluxing. Suitable reaction temperatures are of from 80°C to 250°C, preferably of from 100°C to 200°C, depending in part on the particular diluent employed and the reaction pressure. Suitable reaction pressures are sufficient to maintain the reaction mixture in a liquid phase and vary of from 1 atmosphere to 20 atmospheres, preferably of from 1 atmosphere to 5 atmospheres. Subsequent to reaction the product mixture is separated and the dihydric spirodilactam product is recovered by conventional methods such as precipitation, selective extraction or distillation.

The conversion of the dihydric spirodilactam to the glycidyloxyphenyl-substituted epoxide is conducted in the liquid phase at relatively elevated temperature. The epihalohydrin should be employed in a quantity of at least 2 moles per mole of the dihydric spirodilactam because of the 2:1 stoichiometry of the reaction and preferably the epihalohydrin is employed in a quantity of at least 4 moles per mole of dihydric spirodilactam. Frequently a substantial excess of epihalohydrin is employed to serve as a reaction diluent as well as a reactant. Alternatively, other reaction diluents such as aromatic hydrocarbons including toluene and xylene can be utilised so long as they are relatively inert to the reactants and the epoxy product. Reaction typically is conducted at a temperature of from 80°C to 180°C and a reaction pressure of from 1 atmosphere to 5 atmospheres.

Better results are obtained when the reaction of epihalohydrin and dihydric spirodilactam is conducted in the presence of a quaternary phosphonium salt as catalyst, preferably an alkyltriphenylphosphonium halide. Ethyltriphenylphosphonium iodide or bromide comprise preferred catalysts for the process. The initial reaction product of the epihalohydrin and dihydric spirodilactam is, without isolation, treated with a strong base, typically aqueous sodium hydroxide, while the water both present and formed is removed by distillation under conditions of approximately the normal boiling temperature of the mixture. Such a two-step conversion is entirely conventional for the conversion of hydroxyphenyl-substituted cyclic compounds to the corresponding glycidyloxyphenyl derivative. For example, 2,2-di(4-hydroxyphenyl)propane is converted commercially to the corresponding diglycidyl ether by this technique. Further illustrations of the process are found, for example, in the Encyclopaedia of Polymer Science and Technology, 1968, Vol. 6, 209-222.

Starting from an aromatic dihydric compound ("diphenol") and a di(glycidyloxyphenyl)-substituted compound ("diglycidylether") of which at least a part of either of these compounds is a spirodilactam, a 1,6-diazaspiro[4,4]nonane-2,7-dione derivative can be prepared, that is a linear polyhydric polyether. Preferably the polyhydric polyethers are represented by formula (I), wherein m is larger than 0.

More preferably, p in formula (I) is either 1 or 0, representing an alternating copolymer and a homopolymer respectively. Even more preferably, divalent radical P is a di(oxyphenyl)propane moiety. Still more preferably, P is a 2,2-di(4-oxyphenyl)propane moiety. However, even more preferred 1,6-diazaspiro[4,4]nonane-2,7-dione derivatives are those in which each R is hydrogen, and each n is 0.

It will be apparent that the diphenol and diglycidyl ether will combine in an equimolar relationship to give the polymeric product. Although the ratio of diphenol to diglycidyl ether to be employed are suitably of from 3:1 to 1:3, the reactants are preferably employed in a ratio which is substantially equimolar. Reaction is conducted by mixing the reactants and maintaining the mixture under polymerisation conditions at an elevated temperature. Reaction temperatures to be suitably employed are above 150°C but preferably above 180°C. An atmospheric reaction pressure is useful, although superatmospheric pressures may be utilised. Reactant contact during the reaction is preferably maintained by conventional methods such as shaking or stirring. The reaction conditions, particularly the reaction temperature, will control to some extent the molecular weight of the polyhydric 1,6-diazaspiro[4,4]nonane-2,7 dione derivative. Derivatives of molecular weight of from 10,000 to 100,000 are preferred because of the properties they exhibit.

It is useful on occasion to employ a polymerisation catalyst which is preferably a phosphonium salt, particularly a phosphonium halide although phosphonium acetates and phosphonium bicarbonates are also useful. Such catalysts are conventional in processes of this type and often are alkyltriphenylphosphonium salts. Ethyltriphenylphosphonium salts, particularly ethyltriphenylphosphonium bromide or ethyltriphenylphosphonium iodide are preferred. The phosphonium salt is employed in catalytic quantities. Amounts of the phosphonium salt of up to 5% by mole, based on total reactants, are satisfactory.

Subsequent to reaction the polymeric product is recovered by conventional methods such as precipitation, selective extraction or distillation. The product is often used as such without further purification but the product is purified by conventional techniques such as dissolving the product mixture in a suitable solvent, e.g., an ether such as tetrahydrofuran, and reprecipitating the polymer with an alcohol such as methanol.

The polyhydric polyethers of the invention are characterised by high glass transition temperatures, typically above 150°C or higher. They find utility in the applications conventionally associated with phenoxy resins but additionally are useful for engineering applications such as moulded containers for food and drink which are frequently exposed to elevated temperatures. The polymers can be processed by means of the usual techniques such as injection, compression or blow moulding to prepare films and shaped articles. Moreover, the novel spirodilactam products and the polymers prepared thereof, may be cured using conventional curing agents typically in the art of thermosetting resins.

The invention is further illustrated by the following examples which should not be construed as limiting.

Example I, route A

A mixture of 25 g (0.14 mole) of 4-oxoheptanedioic acid, 31.34 g (0.28 mole) of 4-aminophenol and 100 ml of N,N-dimethylacetamide was placed in a 500 ml round-bottom flask equipped with a mechanical stirrer and a condenser. While being stirred, the mixture was heated to 165°C and refluxed for 12 hours. After cooling, N,N-dimethylacetamide was removed under reduced pressure and methanol was added to precipitate the product. The precipitated product was washed several times with methanol and then dried in a vacuum oven at 150°C for 24 hours. The product had a melting point of 320°C and the nuclear magnetic resonance spectra were consistent with the structure 1,6-di(4-hydroxyphenyl)-1,6-diazaspiro[4.4]nonane-2,7-dione.

Example II, route A

A mixture of 25 g (0.14 mole) of 4-oxoheptanedioic acid, 31.34 g (0.28 mole) of 3-aminophenol and 100 ml of N,N-dimethylacetamide was placed in a 500 ml round-bottom flask equipped with a mechanical stirrer and a condenser. While being stirred, the mixture was heated to 165°C and refluxed for 12 hours. After cooling, the N,N-dimethylacetamide was removed under reduced pressure and methanol was added to precipitate the product. The precipitated product was recovered by filtration, washed several times with methanol and dried in a vacuum oven at 150°C for 24 hours. The product had a melting point of 270°C and the nuclear magnetic resonance spectra were consistent with the structure 1,6-di(3-hydroxyphenyl)-1,6-diazaspiro[4.4]nonane-2,7-dione.

Example III, route B

The procedure of Example I was repeated employing a mixture of 25g (0.16 mole) of 1,6-dioxaspiro[4.4]nonane-2,7-dione and 34.9g (0.32 mole) of 4-aminophenol. The product had a melting point of 320°C and the nuclear magnetic resonance spectra were consistent with the structure 1,6-di(4-hydroxyphenyl)-1,6-diazaspiro[4.4]nonane-2,7-dione.

Example IV, route B

The procedure of Example III was repeated employing 34.9g (0.32 mole) of 3-aminophenol in place of the 4-aminophenol. The product had a melting point of 270°C and the nuclear magnetic resonance spectra were consistent with the structure 1,6-di(3-hydroxyphenyl)-1,6-diazaspiro[4.4]nonane-2,7-dione.

Example V, epoxidation

A mixture of 10.14g (0.03 mole) of 1,6-di(4-hydroxyphenyl)-1,6-diazaspiro[4.4]nonane-2,7-dione, 0.05g of ethyltriphenylphosphonium bromide and 150 ml of epichlorohydrin is placed in a 500 ml round-bottomed flask equipped with a mechanical stirrer and a condenser. The mixture was stirred and heated to 120°C and maintained at 110°C-120°C for four hours. The mixture was then cooled to 80°C-90°C while stirring continued and 5.0 g of 50% aqueous sodium hydroxide solution was added drop-wise as the water present or formed was removed by distillation. After addition of the sodium hydroxide, unreacted epichlorohydrin was removed by distillation under reduced pressure and methanol was added to precipitate the product. The precipitated product was washed several times with methanol and then dried in a vacuum oven for 24 hours. The isolation yield is over 95%. The product had a melting point of 170°C and the nuclear magnetic resonance spectra were consistent with the structure 1,6-di(4-glycidyloxyphenyl)-1,6-diazaspiro[4.4]nonane-2,7-dione.

Example VI, epoxidation

The procedure of Example V was repeated employing a mixture of 10.14g (0.03 mole) of 1,6-di(3-hydroxyphenyl)-1,6-diazaspiro[4.4]nonane-2,7-dione. The product had a melting point of 126°C and the nuclear magnetic resonance spectra were consistent with the structure 1,6-di(3-glycidyloxyphenyl)-1,6-diazaspiro[4.4]nonane-2,7-dione.

Example VII, homopolymerization

A mixture of 2.25g (0.005 mole) of 1,6-di(4-glycidyloxyphenyl)-1,6-diazaspiro[4.4]nonane-2,7-dione, 1.69g (0.005 mole) of 1,6-di(4-hydroxyphenyl)-1,6-diazaspiro[4.4]nonane-2,7-dione and 0.1855g (0.0005 mole) of

ethyltriphenylphosphonium bromide was placed in a reactor of 50 ml capacity equipped with a mechanical stirrer and a condenser. The reaction mixture was heated while being stirred to 200°C and maintained at 200°C for 6 hours. The reaction mixture was then cooled and the reaction product was isolated as a hard resin with a glass transition temperature of 167°C.

Example VIII, homopolymerization

The procedure of Example VII was repeated employing a mixture of 1.69g (0.005 mole) of 1,6-di(3-hydroxyphenyl)-1,6-diazaspiro[4.4]nonane-2,7-dione, and 2.25g (0.005 mole) of 1,6-di(3-glycidyloxyphenyl)-1,6-diazaspiro[4.4]nonane-2,7-dione. The reaction product was isolated as a hard resin with a glass transition temperature of 158°C.

Example IX, copolymerization

The procedure of Example VII was repeated employing a mixture 3.4 g (0.01 mole) of 2,2-di(4-glycidyloxyphenyl)propane, and 3.38 g (0.01 mole) of 1,6-di(4-hydroxyphenyl)-1,6-diazaspiro[4.4]nonane-2,7-dione. The reaction product was isolated as a hard resin with a glass temperature of 157°C.

Example X, copolymerization

The procedure of Example VII was repeated employing a mixture of 1.14g (0.005 mole) of 2,2-di(4-hydroxyphenyl)propane, and 2.25g (0.005 mole) of 1,6-di(4-glycidyloxyphenyl)-1,6-diazaspiro[4.4]nonane-2,7-dione. The reaction product was isolated as a hard resin with a glass transition temperature of 156°C.

Comparative Example

The reaction product of 2,2-di(4-glycidyloxyphenyl)propane and 2,2-di(4-hydroxyphenyl)propane was produced by the procedure of Example VII. The product, a commercial resin, had a glass transition temperature of 87°C.

## Claims

### Claims for the following Contracting States : BE, CH, DE, FR, GB, IT, LI, NL

1. A 1,6-diazaspiro[4,4]nonane-2,7-dione derivative having the formula $X-[-B-Y-(P-Y)_p-]_m-B-X$, in which: B is a divalent radical of formula (I),

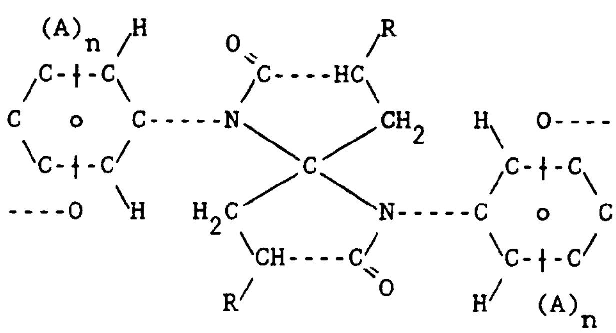

(I)

wherein R independently is hydrogen or a $C_{1-4}$ alkyl, A is a substituent replacing hydrogen selected independently from a $C_{1-4}$ alkyl or halogen atom, and n independently is 0, 1 or 2; m ≧ 0; each X independently is hydrogen or a glycidyl group; Y is 2-hydroxy-1,3-propadiyl; each P independently is a divalent radical derived from a dihydric aromatic alcohol; and p is on average of from 0 to 3.

2. A 1,6-diazaspiro[4,4]nonane-2,7-dione derivative as claimed in claim 1, wherein p is 0 or 1.

3. A 1,6-diazaspiro[4,4]nonane-2,7-dione derivative as claimed in claim 1 or 2, wherein m is selected such that the the derivative has a molecular weight of from 10,000 to 100,000.

4. A 1,6-diazaspiro[4,4]nonane-2,7-dione derivative as claimed in claim 1, 2 or 3, wherein R is methyl or hydrogen.

5. A 1,6-diazaspiro[4,4]nonane-2,7-dione derivative as claimed in any of claims 1 to 4, wherein A is methyl.

6. A 1,6-diazaspiro[4,4]nonane-2,7-dione derivative as claimed in any of claims 1 to 5, wherein R is hydrogen, and n is 0.

7. A 1,6-diazaspiro[4,4]nonane-2,7-dione derivative as claimed in any of claims 1 to 6, wherein divalent radical P is derived from 2,2-di(4-hydroxyphenyl)propane.

8. A 1,6-diazaspiro[4,4]nonane-2,7-dione derivative as claimed in any of claims 1 to 6, wherein m is 0, and each X is either hydrogen or a glycidyl group.

**Claims for the following Contracting State : ES**

1. Process for the preparation of 1,6-diazaspiro-[4,4]nonane-2,7-dione derivatives having the formula $X\text{-}\{\text{-}B\text{-}Y\text{-}(P\text{-}Y)_p\text{-}\}_m\text{-}B\text{-}X$, in which:
B is a divalent radical of formula (I),

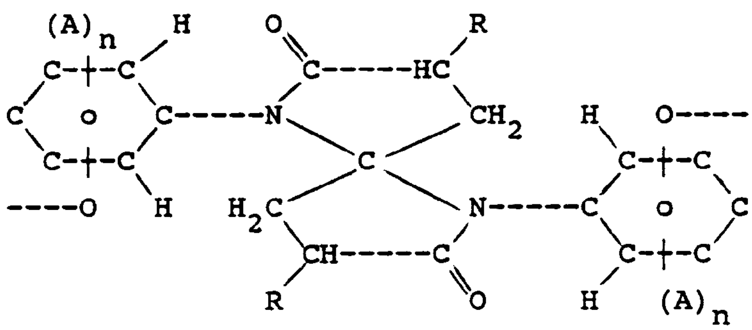

(I)

wherein R independently is hydrogen or a $C_{1-4}$ alkyl, A is a substituent replacing hydrogen selected independently from a $C_{1-4}$ alkyl or halogen atom, and n independently is 0, 1 or 2; $m \geqq 0$; each X independently is hydrogen or a glycidyl group; Y is 2-hydroxy-1,3-propadiyl; each P independently is a divalent radical derived from a dihydric aromatic alcohol; and p is on average of from 0 to 3, which comprises reacting in liquid phase solution an aminophenol of formula (II),

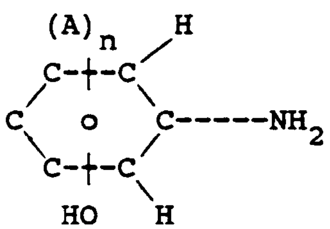

(II)

wherein A and n have the same meaning as defined above, with either (reactant IIa) a 4-oxoheptanedioic acid compound of formula (III),

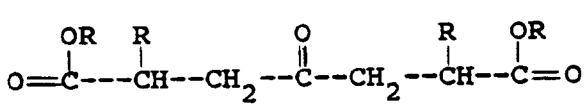

(III)

or (reactant IIb) a 1,6-dioxaspiro[4,4]nonane-2,7-dione of formula (IV),

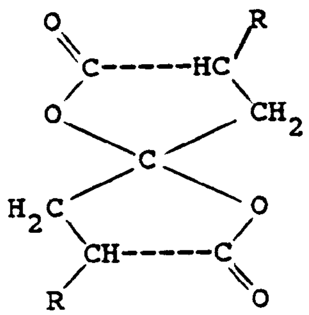

(IV)

wherein R for both reactants has the same meaning as defined above; followed, when m is greater than zero, by epoxidation.

2. Process as claimed in claim 1, wherein the aminophenol is employed in amount of from 1.5 mole to 3 moles per mole of reactant II.

3. Process as claimed in claim 1 or 2, wherein the reaction takes place in a solvent selected from dialkyl ketones, esters, ethers, or N,N-dialkylamides.

4. Process as claimed in claim 1, 2 or 3, wherein the reaction is carried out at a temperature between 100°C and 200°C.

5. Process as claimed in any one of the preceeding claims, wherein the dihydric spirodilactam product (m=O) is epoxidised to a glycidyloxyphenyl-substituted epoxide by reaction with an epihalohydrin in the presence of a quaternary phosphonium salt as catalyst.

**Patentansprüche**

**Pantentansprüche für folgende Verstragsstaaten : BE, CH, DE, FR, GB, IT, LI, NL**

1. Ein 1,6-Diazaspiro[4,4]nonan-2,7-dionderivat der Formel X-{B-Y(P-Y)$_p$-}$_m$-B-X, in welcher B ein zweiwertiges Radikal der Formel (I) ist,

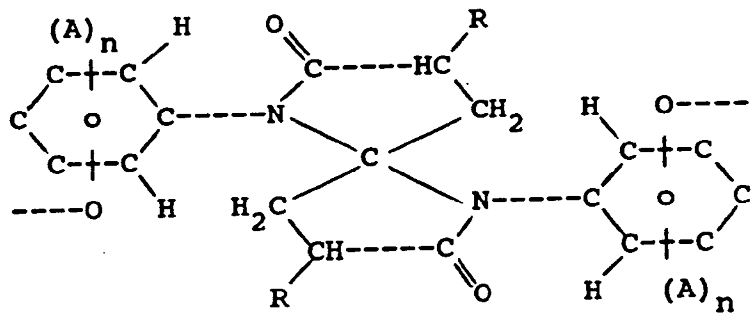

(I)

in welcher R unabhängig Wasserstoff oder eine $C_{1-4}$-Gruppe ist, A ein Substituent ist, der den Wasserstoff ersetzt und unabhängig ausgewählt ist aus einem $C_{1-4}$-Alkyl oder einem Halogenatom, und n unabhängig den Wert 0, 1 oder 2 hat; m ≧ 0; X jeweils unabhängig Wasserstoff oder eine Glycidylgruppe ist; Y 2-Hydroxy-1,3-propadiyl ist; P jeweils unabhängig ein zweiwertiges Radikal ist, abgeleitet von einem zweiwertigen aromatischen Alkohol; und p durchschnittlich einen Wert von 0 bis 3 hat.

2. Ein 1,6-Diazaspiro[4,4]nonan-2,7-dionderivat wie in Anspruch 1 beansprucht, in welchem p 0 oder 1 ist.

3. Ein 1,6-Diazaspiro[4,4]nonan-2,7-dionderivat wie in Anspruch 1 oder 2 beansprucht, in welchem m so gewählt ist, daß das Derivat ein Molekulargewicht von 10000 bis 100000 hat.

4. Ein 1,6-Diazaspiro[4,4]nonan-2,7-dionderivat wie in Anspruch 1, 2 oder 3 beansprucht, in welchem R Methyl oder Wasserstoff ist.

5. Ein 1,6-Diazaspiro[4,4]nonan-2,7-dionderivat wie in einem der Ansprüche 1 bis 4 beansprucht, in welchem A Methyl ist.

6. Ein 1,6-Diazaspiro[4,4]nonan-2,7-dionderivat wie in einem der Ansprüche 1 bis 5 beansprucht, in welchem R Wasserstoff ist und n den Wert 0 hat.

7. Ein 1,6-Diazaspiro[4,4]nonan-2,7-dionderivat wie in einem der Ansprüche 1 bis 6 beansprucht, in welchem das zweiwertige Radikal P abgeleitet ist von 2,2-Di(4-hydroxyphenyl)propan.

8. Ein 1,6-Diazaspiro[4,4]nonan-2,7-dionderivat wie in einem der Ansprüche 1 bis 6 beansprucht, in welchen m den Wert 0 hat und X jeweils entweder Wasserstoff oder eine Glycidylgruppe ist.

**Pantentansprüche für folgenden Verstragsstaat : ES**

1. Verfahren zur Herstellung von 1,6-Diazaspiro[4,4]nonan-2,7-dionderivaten der Formel $X\{B-Y\{P-Y\}_p\}_m B-X$, in welcher B ein zweiwertiges Radikal der Formel (I) ist

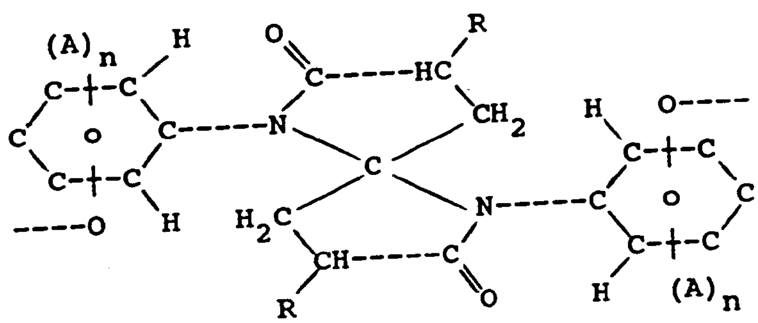

(I)

in welcher R unabhängig Wasserstoff oder eine $C_{1-4}$-Alkylgruppe ist, A ein Substituent ist, der Wasserstoff ersetzt und unabhängig ausgewählt ist aus einem $C_{1-4}$-Alkyl oder einem Halogenatom, und n unabhängig den Wert 0,1 oder 2 hat; $m \geqq 0$ ist , X jeweils unabhängig Wasserstoff oder eine Glycidylgruppe ist; Y 2-Hydroxy-1,3-propadiyl ist; P jeweils unabhängig ein zweiwertiges Radikal, abgeleitet von einem zweiwertigen aromatischen Alkohol, ist; und p durchschnittlich 0 bis 3 ist, welches Verfahren das Umsetzen in Flüssigphasenlösung eines Aminophenols der Formel(II),

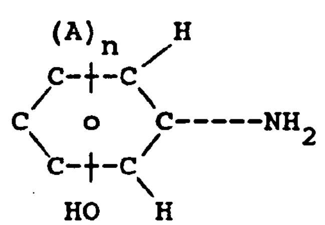

(II)

in welcher A und n die oben genannten Bedeutungen haben, mit entweder(Reaktand IIa) einer 4-Oxopheptandicarbonsäureverbindung der Formel(III)

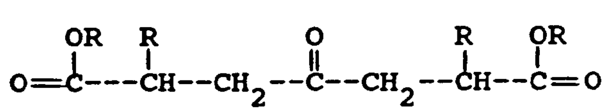

(III)

oder (Reaktand IIb) einem 1,6-Dioxaspiro(4,4(nonan-2,7-dion der Formel(IV), umfaßt

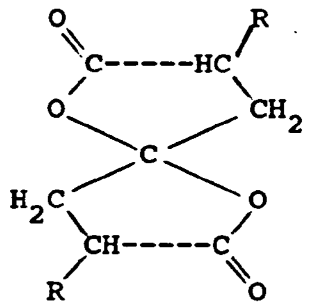

(IV)

wobei R in beiden Reaktanden die gleiche Bedeutung wie oben angegeben haben, woran sich, wenn m einen Wert von größer als 0 hat, eine Epoxidation anschließt.

2. Verfahren wie in Anspruch 1 beansprucht, in welchem das Aminophenol in einer Menge von 1,5 Mol bis 3 Mol je Mol des Reaktanden II eingesetzt wird.

3. Verfahren wie in Anspruch 1 oder 2 beansprucht, in welchem die Reaktion in einem Lösungsmittel, ausgewählt aus Dialkylketonen, Estern, Äthern oder N,N-Dialkylamiden, stattfindet.

4. Verfahren wie in Anspruch 1, 2 oder 3 beansprucht, in welchem die Reaktion bei einer Temperatur zwischen 100°C und 200°C stattfindet.

5. Verfahren wie in einem der vorstehenden Ansprüche beansprucht, in welchem das zweiwertige Spirodilactamprodukt(m=O) durch Umsetzung mit einem Epihalohydrin in Gegenwart eines quartären Phosphoniumsalzes als Katalysator zu einem mit Glycidyloxyphenyl substituierten Epoxid epoxidiert wird.

## Revendications

**Revendications pour les Etats contractants suivants : BE, CH, DE, FR, GB, IT, LI, NL**

1. Dérivé de 1,6-diazaspiro[4,4]nonane-2,7-dione de formule X-[-B-Y-(p-Y)$_p$]$_m$-B-X dans laquelle :
B est un radical bivalent de formule (I) :

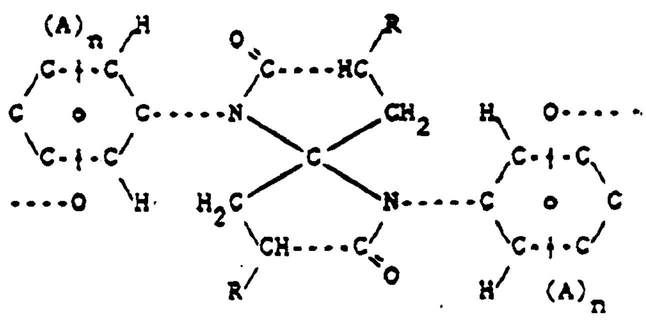

(I)

dans laquelle R représente indépendamment un hydrogène ou un alcoyle en $C_{1-4}$; A est un substituant remplaçant l'hydrogène choisi indépendamment parmi un alcoyle en $C_{1-4}$ ou un atome d'halogène, et n vaut indépendamment 0, 1 ou 2 ; m ≧ 0 ; chaque X représente indépendamment un hydrogène ou un groupe glycidyle ; Y est un 2-hydroxy-1,3-propadiyle ; chaque P représente indépendamment un radical bivalent dérivé d'un alcool aromatique dihydrique ; et p vaut en moyenne de 0 à 3.

2. Dérivé de 1,6-diazaspiro[4,4]nonane-2,7-dione selon la revendication 1, où p vaut 0 ou 1.

3. Dérivé de 1,6-diazaspiro[4,4]nonane-2,7-dione selon la revendication 1 ou 2, où m est choisi de manière que le dérivé ait un poids moléculaire allant de 10 000 à 100 000.

4. Dérivé de 1, 6-diazaspiro [4,4] nonane-2, 7-dione selon la revendication 1, 2 ou 3, où R est un méthyle ou un hydrogène.

5. Dérivé de 1,6-diazaspiro[4,4]nonane-2,7-dione selon l'une quelconque des revendications 1 à 4, où A est un méthyle.

6. Dérivé de 1,6-diazaspiro[4,4]nonane-2,7-dione selon l'une quelconque des revendications 1 à 5, où R est un hydrogène et n vaut 0.

7. Dérivé de 1,6-diazaspiro[4,4]nonane-2,7-dione selon l'une quelconque des revendications 1 à 6, où le radical bivalent P est dérivé du 2,2-di(4-hydroxyphényl)propane.

8. Dérivé de 1,6-diazaspiro[4,4]nonane-2,7-dione selon l'une quelconque des revendications 1 à 6 où m vaut 0, et chaque X représente un hydrogène ou un groupe glycidyle.

**Revendications pour l'Etat contractant suivant : ES**

1. Procédé de préparation de dérivés de 1,6-diazaspiro [4,4]nonane-2,7-dione ayant la formule X-[-B-Y-(P-Y)$_p$]$_m$-B-X dans laquelle :
B est un radical bivalent de formule (I) :

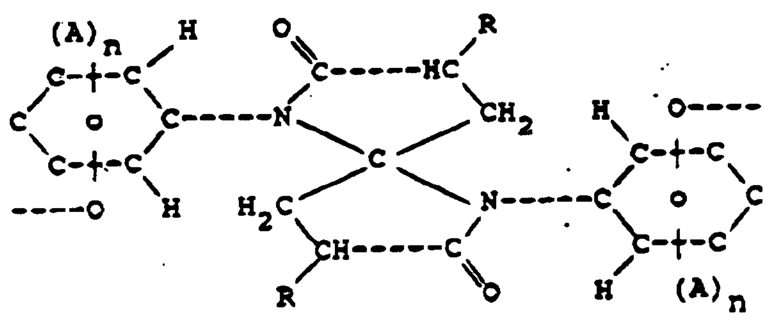

(I)

dans laquelle R représente indépendamment un hydrogène ou un alcoyle en $C_{1-4}$, A est un substituant remplaçant l'hydrogène choisi indépendamment parmi un alcoyle en $C_{1-4}$ ou un atome d'halogène, et n vaut indépendamment 0, 1 ou 2 ; m $\geqq$ 0 ; chaque X représente indépendamment un hydrogène ou un groupe glycidyle ; Y est un 2-hydroxy-1,3-propadiyle ; chaque P représente indépendamment un radical bivalent dérivé d'un alcool aromatique dihydrique ; et p vaut en moyenne de 0 à 3, dans lequel on fait réagir dane une solution en phase liquide un aminophénol de formule (II),

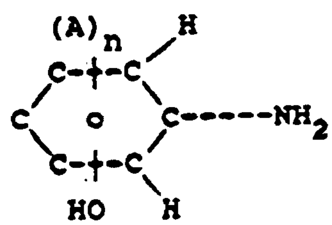

(II)

dans laquelle A et n ont la même signification que définie ci-dessus, avec soit (réactif IIa) un composé d'acide 4-oxoheptanedioïque de formule (III),

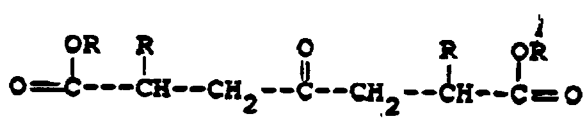

(III)

soit (réactif IIb) une 1,6-dioxaspiro[4,4]nonane-2,7-dione de formule (IV),

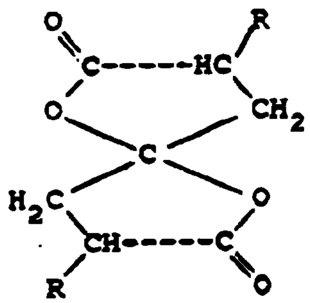

(IV)

où R pour les deux réactifs à la même signaification que définie ci-dessus ; puis, lorsque m est supérieur à 0, on procède à une époxydation.

2. Procédé selon la revendication 1, dans lequel l'aminophénol est employé en une quantité allant de 1,5 mole à 3 moles par mole de réactif II.

3. Procédé selon la revendication 1 ou 2, dans lequel la réaction se déroule dans un solvant choisi parmi les dialcoyl-cétones, les esters, les éthers ou les N,N-dialcoylamides.

4. Procédé selon la revendication 1, 2 ou 3, dans lequel la réaction est conduite à une température entre 100°C et 200°C.

5. Procédé selon l'une quelconque des revendications précédentes, dans lequel le produit spirodilactame dihydrique (m=0) est époxydé en un époxyde glycidyloxyphényl-substitué par réaction avec une épihalohydrine en présence d'un sel de phosphonium quaternaire comme catalyseur.